# EUROPEAN PATENT SPECIFICATION

(11) **EP 2 464 252 B1**
(45) Date of publication and mention of the grant of the patent: **29.04.2020**
(21) Application number: 09848320.9
(22) Date of filing: 13.08.2009
(51) Int. Cl.: A41D 13/05, A61F 5/449, A61F 5/457

(54) **BACK SUPPORT GARMENT APPARATUS**
KLEIDUNGSSTÜCK MIT RÜCKENSTÜTZE
APPAREIL FORMANT VÊTEMENT DE SUPPORT DORSAL

(43) Date of publication of application: 20.06.2012
(73) Proprietor: Arsenault, James, East Williston, NJ 11596 (US); Iskyan, Paul, Manhasset, NJ 11030 (US); Vad, Vijay, New York, NY 10021 (US)
(72) Inventor: Arsenault, James, East Williston, NJ 11596 (US); Iskyan, Paul, Manhasset, NJ 11030 (US); Vad, Vijay, New York, NY 10021 (US)
(74) Representative: Staudt, Armin Walter
(86) International application number: PCT/US2009/004630
(87) International publication number: WO 2011/019332

(56) References cited:
- EP-B1- 0 636 325
- CN-Y- 201 219 940
- GB-A- 2 259 848
- JP-A- 2004 229 783
- US-A- 5 157 790
- US-A- 5 205 815
- US-A- 5 722 940
- US-A- 6 108 819
- US-A1- 2005 268 379

## Description

### Field of the Invention

The present invention relates to back support garments, and more particularly to a one-piece support belt and compression pants apparatus.

### Background of the Invention

Millions of Americans suffer from back injuries and back pain, which can easily be aggravated by participation in athletic, physical, and even everyday activities. The lower back, or lumbar region, supports the weight of the upper body and is the most common site of back injuries. Treatment for back injuries often involves restoring strength to the back and preventing recurrence of the injury.

Back patients often wear back support garments to compress and restrict movement in the lumbar spine and surrounding muscles to prevent further back strain. There are many variations of compressive back supports in the prior art. These often consist of a back support device made from a stiff fabric configured to compress the wearer's waist area.

Although several back supports exist in the prior art, most existing back support apparatuses provide compressive support only to the lumbar area, and fail to extend support to the tailbone region of the spine and its surrounding muscles, which are also vulnerable to injury. Furthermore, back supports of the prior art also do not provide adequate compressive support to a wearer's leg and groin muscles. In addition, they do not provide for localized therapeutic heating or cooling of the lumbar region.

U.S. 5,205,815 A shows a garment with compression pants attached to support band and at least one pair of tapered side pull elastic adjustable strap members. Each of the strap members is stitched with the support band and with one of its ends in alignment with a centrally positioned stay member. The stay member is in direct vertical alignment with the spinal column of the wearer. The stitching of the strap members almost completely overlies the rear portion of the shorts. The strap members do not extend upwardly from the compression pants.

GB 2 259 848 B shows a garment with a belt that is connected with pants by snaps or Velcro®. The belt is not fixedly attached to the pants but connected with snaps. The belt also is not a support for the wearer, but actually just a protective armor plate for the wearer's kidneys.

EP 0 636 325 B1 shows a garment with a closed tubular belt structure attached to a garment. The belt structure has a stretchable auxiliary belts extending upward from the leg pants. The auxiliary belts are placed between the face cloth and the lining cloth and do not possess any opening and closing means. The belt structure is attached fixedly to and extending upwardly from the leg pants so as to align the rear portion with the lumbar region of the wearer,

### Summary of the Invention

It is accordingly an object of the present invention to provide a back support apparatus that does not have the drawbacks of the prior art.

An object of the present invention is to provide a one-piece apparatus that evenly distributes forces applied on the body during athletic, physical and every day activity, in particular those applied during twisting or forward bending movements, such as when swinging a golf club or skiing downhill, over a wearer's lumbar to mid-thigh regions, while simultaneously providing therapeutic heating and cooling benefits.

In accordance with an aspect of the present invention, a back support garment for a wearer includes a compression pants portion configured to provide support for the wearer's pelvis, legs, and groin area. A belt portion attached fixedly to and extending upwardly from the compression pants portion and has a rear portion configured to support to a lumbar and an abdominal area of the wearer. The belt portion is attached to the pants portion so as to align its rear portion with the lumbar region of the wearer. The belt portion comprises a first end and a second end that are releasably attachable to each other so as to adjustably secure said belt portion tightly around the wearer's waist.

The back support garment has a pack support structure with one or more slots supporting therein one or more hot or cold packs adjacent an inner surface of the pack support structure. The belt portion is attached to the pants portion so as to align the pack support portion with a lumbar region of the wearer and to maintain temperature transferring contact between the inner surface of the belt portion adjacent the hot and cold packs and the wearer's lumbar region. The rear portion of said compression pants portion is fixedly secured to the rear portion of said belt portion adjacent the hot or cold packs.

According to an aspect of the invention, the compression pants portion is shorts extending no lower than the thighs of the wearer. In another aspect of the invention, the compression pants portion extends past the wearer's knees.

In another aspect of the invention, a rear portion of the belt portion has an insert receiving structure with one or more slots supporting therein one or more inserts configured to provide further support for the wearer's back.

Other objects and advantages of the invention herein will become apparent in the specification below.

### Brief Description of the Drawings

FIG. 1 is a front view of an embodiment of the support garment of the invention, as worn by a user.
FIG. 2 is a back view of the support garment of FIG 1.
FIG. 3 is a left-hand side view of the support garment of FIGS. 1 and 2.
FIG. 4 is a front view of the garment of FIGS. 1-3 when not worn, showing the pack support structure having multiple slots for receiving hot or cold packs.
FIG. 5 is a back view of the garment as seen in FIG 4.
FIG. 6 is a front view of an alternate embodiment of the present invention, as worn by a user.
FIG. 7 is an enlarged partial front view of another alternate embodiment of the present invention, showing the showing the pack support structure having only one slot for receiving hot or cold packs.

### Detailed Description

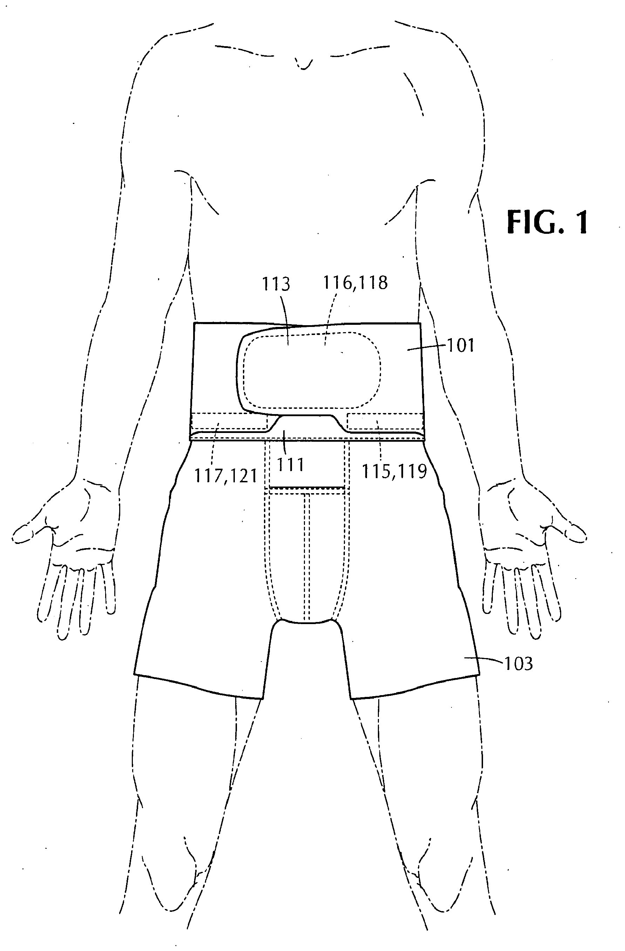

As best seen in Figures 1, 2 and 3, the present apparatus generally comprises a unitary garment having a belt portion 101 and a compression pants portion 103. Belt portion 101 encircles the waist of a wearer, and extends upwardly from the compression pants portion 103.

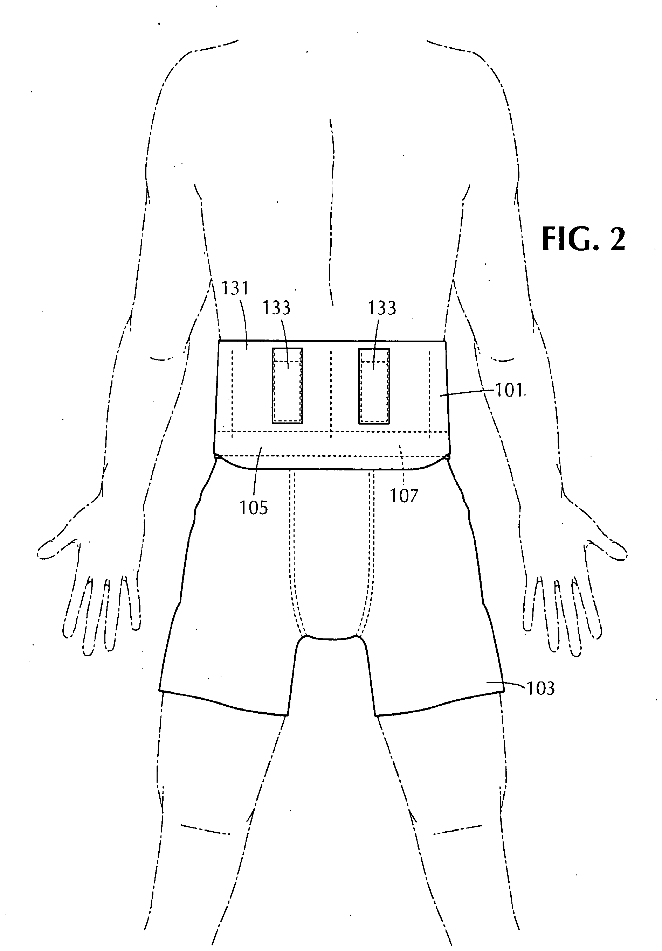

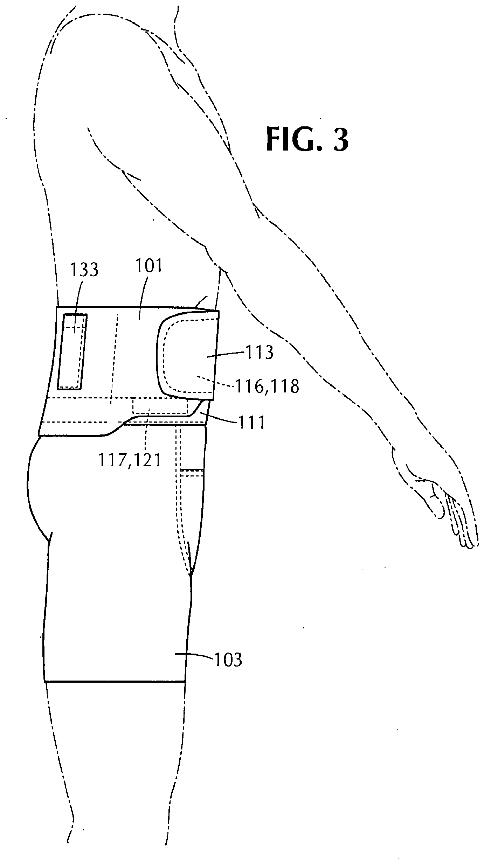

As shown in Figures 2 and 3, the rear portion 105 of the belt portion 101 is fixedly secured to a rear portion 107 of the pants portion 103, thus locating the belt of the user when wearing the pants portion 103, and preventing separation of the pants portion 103 from the belt portion 101 when the wearer moves. Securing the pants portion 103 and the belt portion 101 distributes the compressive support provided by the pants and the belt individually over the entire lumbar region extending from the wearer's waist to the wearer's tailbone area.

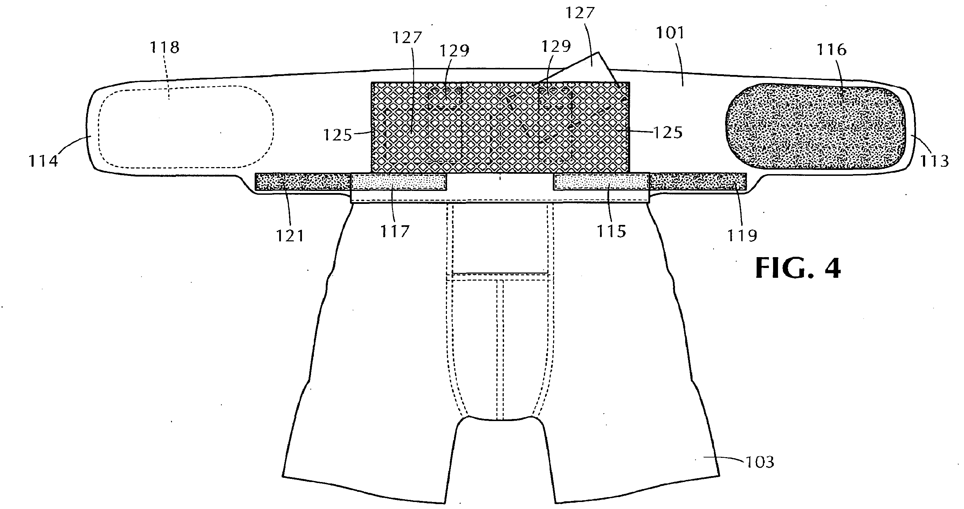

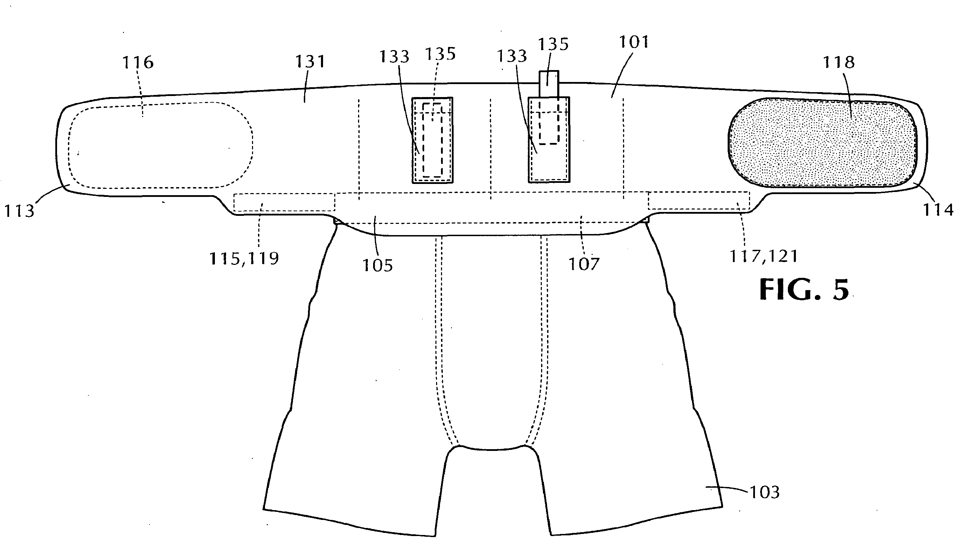

Referring to Figure 5, the rear portion 107 of the pants portion 103 and the rear portion 105 of the belt portion 101 are fixedly secured to each other, as by glue or stitching, while, as shown in Figure 4, the forward waistband portion 111 of the pants portion 103 and the forward portion 113 of the belt portion 101 are configured so as to be releasably attachable to one another by releasable connection structures or means. These portions 111 and 113 are adjustably and releasably connected with each other so that the user can adjust a relative position of the forward waistband portion 111 of the pants portion 103 in relation to the forward portion 113 of the belt portion 101, so as to comfortably conform the garment to the wearer's body.

In the embodiment depicted in Figure 4, the forward waistband portion 111 of the pants portion 103 has secured thereon one or more strips of a hook and loop fastening fabric strips 115 and 117, such as Velcro®. The unsecured bottom portions of the belt portion 101 are lined with complementary strips of hook and loop fastening fabric strips 119 and 121, respectively, extending circumferentially about the waist of the user and that releasably secure the forward portion 113 of belt portion 101 to forward portion 111 of pants portion 103, by co-acting securement of fabric strips 115 and 117 with fabric strips 119 and 121.

The compression pants portion 103 is constructed from a light-weight elastic material having the requisite stiffness and elasticity to compressively support the abdominal, groin and leg muscles of the wearer, while also being comfortable and providing ease of movement during athletic, physical or daily activity. Examples of materials which are suitable for these purposes include fabrics containing Lycra, Spandex, or a similar stretch material. In the preferred embodiment of the invention, the material further has moisture-wicking capabilities which further prevent chafing and allow for cooling of the covered muscles when the user is performing sweat-inducing activities.

In the preferred embodiment, the compression pants portion 103 is shorts that extend no lower than the thighs of the wearer, from the wearer's waist area to a mid or lower thigh position. This length provides compressive support to the wearer's abdominal, groin, and quadriceps muscles, and is suitable for wear during most athletic, physical, or everyday activities.

The belt portion 101 is constructed from a stiff fabric to enhance stabilization and support of the lumbar region, while also allowing the wearer enough freedom of movement to engage in athletic, physical or daily activity requiring twisting or bending of the spine. Synthetic laminated or woven stretchable fabrics, such as Neoprene, manufactured by the DuPont Corporation, are desirable due to their stiffness, flexibility, and insulating properties. In the preferred embodiment of the invention, the material is a permeable or breathable fabric that also wicks perspiration away from the skin for enhanced comfort, such as Breathoprene ®, by AccuMED Technologies, Inc. The material forming the belt portion is sufficiently thin so as to make the belt invisible when worn under other garments or athletic attire. Preferably, the overall thickness of the belt is between 1 mm and 5mm, as this provides the requisite amount of lumbar support, while maintaining the invisibility of the belt under the wearer's outer garments.

Use of the above materials is desirable for their mechanical properties, but such material may cause sticking of the wearer's outer garments to the belt. Accordingly, the outer surface of the belt is covered by a thin, smooth fabric such as Nylon so as to minimize friction between the user's outer clothing and belt during periods of contact, and to prevent bunching of the wearer's outer garments around the belt. The fabric covering the outer surface of the belt should be so thin that it has no, or minimal effect on the overall thickness of the belt.

As best seen in Figures 4 and 5, belt portion 101 has a first and second ends 113 and 114, that are each configured so as to be releasably attachable to each other, allowing the wearer to fasten the belt portion 101 tightly around the wearer's waist in a range of possible waist sizes, so as to wear the belt snugly as depicted in Figures 1, 2 and 3. The first end 113 of the belt portion 101 has secured thereon a patch 116 of hook and loop type fastening fabric, and the second end 114 of the belt portion 101 is lined with a complementary co-acting patch 118 of hook and loop type fastening fabric. The patches 116 and 118 are large enough about the waist of the wearer, and configured to be releasably secured to each other in a variety of waist size positions and with some varying angulation, if desired. Alternatively, the entire inner surface 123 of the belt can be lined with the loop material so as to co-act with a patch of hook fabric secured onto the second end 114 of the belt. Other types of fastening mechanisms, such as a buckle or lace-up configuration having openings in it may also be used to adjustably secure the belt portion 101 around the waist of the user.

As shown in Figure 4, the inner surface 123 of the belt portion 101 includes two pack support structures 125 with one or more slots therein configured to receive therapeutic hot or cold packs 127. The pack support structures 125, which are secured to the inner surface 123 of the belt portion 101 by glue or stitching, are positioned so as to maintain temperature transferring contact between the inner fabric of the support structures 125 adjacent the inserted therapeutic packs 127 and the lumbar region of the wearer when.the belt portion 101 is fastened around the wearer's waist. Preferably, the therapeutic packs 127 are sized so as to cover the wearer's entire back waist region when inserted into the slots, including the spinal cord and its surrounding muscles.

The pack support structures 125 are preferably formed from a single piece of waterproof and breathable material such as nylon, which is sufficiently strong to accommodate the weight of the pack without tearing, but which is thin enough so that the hot or cold effects of the packs can instantly be felt by the wearer. A mesh material, as shown, may be employed for the inner fabric of the support structures 125, or a piece of continuous material may be used.

As shown in Figure 4, the inner fabric of the pack support structures 125 and the inner surface 123 of the belt portion 101 adjacent the therapeutic packs 127 have secured thereon strips of co-acting hook and loop fastening fabric 129, thereby allowing the wearer to close the openings formed by the pack support structures 125 and firmly position the gel packs 125 in the pack support structures. Other types of closure mechanisms, such snaps or buttons, may also be used to close the openings formed by the pack support structures.

A wide variety of therapeutic hot and cold packs are commercially available for use with the present invention. Ice packs, for example, are often distributed as pre-sealed plastic sacks containing refrigerant gels or liquids, but can also be homemade variants made from suitable plastic bags filled with crushed or cubed ice. Heat packs are also widely available as microwavable plastic sacks containing a liquid or a gel with a high specific heat. Commercially available electric heating and cooling packs may also be used.

As best shown in Figure 5, the outer surface 131 of the rear portion 105 of the belt portion 101 is provided with one or more additional insert support structures 133 secured fixedly thereon and configured to removably receive one or more rigid inserts 135. Preferably, an insert support structure 133 is positioned in the belt portion 101 so that when worn, the inserts 135 each align spaced on each side adjacent the user's spinal cord so that the inserts 135, when placed in the insert support structures 133, provide additional support to the spinal cord and its surrounding muscles, or help the wearer maintain proper back alignment.

A user may choose not to use inserts 135 with the slots in pack support structures 133, since the inserts 135 further restrict the range of movement of the user's spine, potentially making it difficult for the wearer to engage in certain athletic, physical or everyday activities. This embodiment relying on the inserts may be desirable for individuals nursing a more serious back injury requiring extra support. The removable nature of the rigid inserts 135 means that the support garment can be selectively used with or without support, depending on the specific requirements of the selected activity of the user.

The inserts 135 are formed from a lightweight material, such as plastic or rubber, and have a variable resistance to bending that is determined by the insert's thickness and the properties of the material from which the insert 135 is formed. The inserts 135 are sufficiently thin so as to be less visible when the garment is worn under other clothing, and are of a sufficient length so as to extend over the lumbar region of the wearer extending above the pelvis. The insert support structures 133 receiving the inserts 135 are each preferably formed from a single piece of material having sufficient strength to accommodate the weight of the insert 135, and to secure the insert 135 in stiffening support of the belt portion 101.

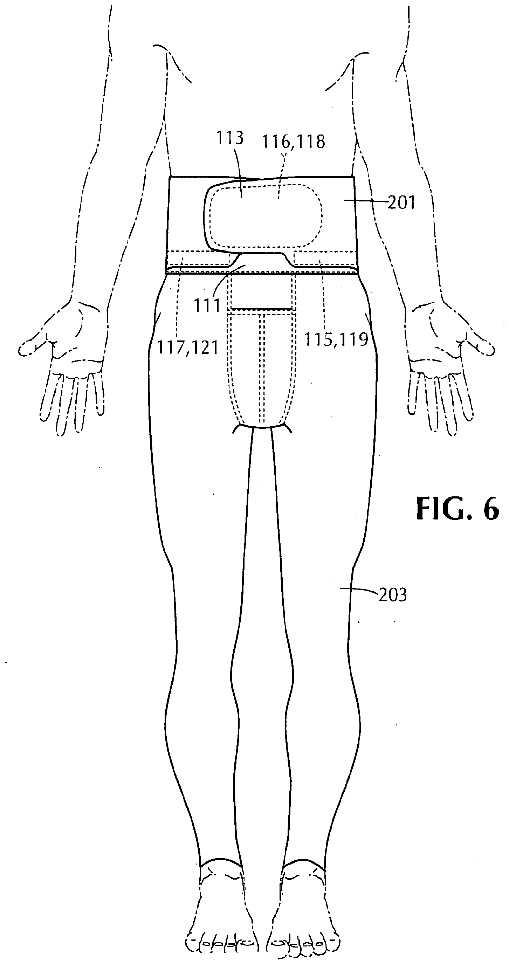

In an alternate embodiment, which is depicted in Figure 6, compression pants portion 203 extends downward past the wearer's lower thigh, so as to also cover a user's knees and calves. This embodiment also provides added warmth and support to a wearer's calf muscles, and is preferable for wear during cold weather activities, such as skiing, skating, or snowmobiling. Belt portion 201 is configured similarly to the embodiment of Figures 1 to 5 and the same reference numbers are used for corresponding parts thereof.

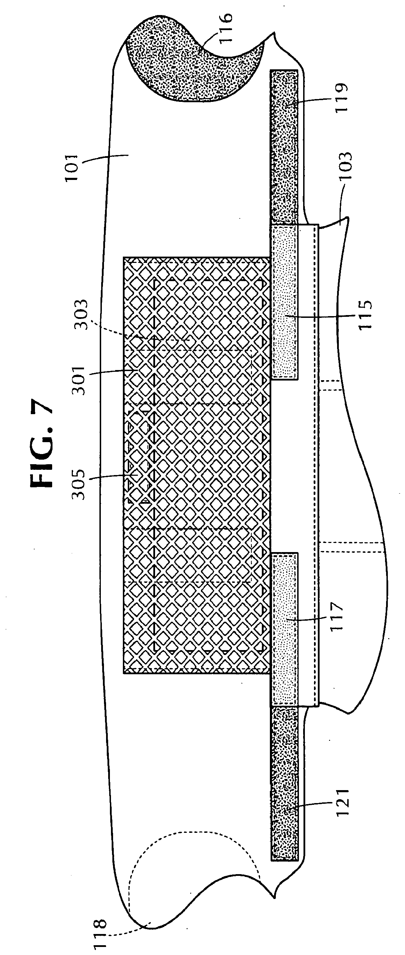

In a further alternate embodiment of the invention, shown in Figure 7, the pack support structure 301 has a single interior space extending laterally substantially across the back of the user. This structure 301 is configured to receive and support therein a single, elongated therapeutic hot or cold pack 303 that is sized so as to cover the wearer's entire back waist region in the interior space. The inner fabric of the pack support structure 301 and the inner surface 123 of the belt portion 101 adjacent the elongated hot or cold pack 303 are releasably secured to each other by strips of co-acting hook and loop fastening fabric 305 thereon, so as to allow the wearer to close the space 303.

The materials and construction of the belt 101 is otherwise similar to that of the belt portion 101 of the preferred embodiment, and similar reference characters are used for complementary parts.

## Claims

1. A back support garment for a wearer, said garment comprising:
a compression pants portion (103) being configured to provide support for the wearer's pelvis, legs, and groin area; and
a belt portion (101) being configured to support a lumbar and an abdominal area of the wearer, and including a rear portion (105) configured to overlay and
support the lumbar portion of the wearer;
said belt portion (101) comprises a first end (113) and a second end (114) that are releasably attachable to each other so as to adjustably secure said belt portion (101) tightly around the wearer's waist;
wherein said belt portion (101) is attached fixedly to and extending upwardly from said compression pants portion (103) so as to align the rear portion (105) with the lumbar region of the wearer,
wherein the back support garment has a pack support structure (301) supporting therein one or more hot or cold packs (303)
**characterized in that**
the pack support structure (301) is adjacent an inner surface of said belt portion (101),
said belt portion (101) being attached to said pants portion (103) so as to align the pack support portion (301) with the lumbar region of the wearer and to maintain temperature transferring contact between an inner surface of the pack support structure (301) adjacent said hot and cold packs (303) and the wearer's lumbar region, and
wherein a rear portion (107) of said compression pants portion (103) is fixedly secured to the rear portion (105) of said belt portion (101) adjacent the hot or cold packs (303).

2. The back support garment of claim 1, wherein said rear portion (105) of the belt portion (101) includes rigidifying structure comprising an insert receiving structure (133) supporting therein one or more inserts (135) being configured to provide further support for the wearer's back, said inserts (135) being composed of material having a resistance to bending greater than that of the rear portion (105) of the belt portion (101), wherein said insert support structure (133) has slots receiving said inserts (135), and said inserts (135) being removable by the wearer by sliding out of the associated slot, and wherein said inserts (135) are plastic.

3. The back support garment according to claim 1 or 2, wherein said compression pants portion (103) is shorts extending no lower than the thighs of the wearer, or said compression pants portion (103) extends downwardly past the wearer's knees.

4. The back support garment according to claim 1, wherein first and second ends (113, 114) of said belt portion (101) are arranged in a front portion of said belt portion (101).

5. The back support garment according to claim 4, wherein said first end (113) of said belt portion (101) has hook and loop fastener material (116) fixedly secured thereto and said second end (114) of said belt portion has complementary hook and loop fastener material (118) fixedly secured thereto so that said first end (113) is releasably and adjustably attachable to said second end (114).

6. The back support garment according to one or more of the preceding claims, wherein said pants portion (103) has a forward waistband portion (115, 117) that is releasably attachable to a portion (119, 121) of said belt portion (101) to adjust a relative position of the pants portion (103) to the belt portion (101).

7. The back support garment according to claim 6, wherein said forward waistband portion (115, 117) of said pants portion (103) has hook and loop fastener fabric fixedly secured thereto and said portion of said belt portion (101) has complementary hook and loop fastener material (119, 121) fixedly secured thereto to adjust a relative position of the pants portion (103) to the belt portion (101).

8. The back support garment according to one or more of the preceding claims, wherein said belt portion (101) is made from a breathable and semi-rigid material.

9. The back support garment according to one or more of the preceding claims, wherein said compression pants portion (103) is made from a material containing an elastic fabric so that the compression pants portion (103) compresses the wearer's body when thereon, wherein said compression pants portion (103) is made is made from a material having moisture-wicking properties, and wherein said compression pants portion (103) is made is made from nylon fabric.

10. The back support garment according to one or more of the preceding claims, wherein surfaces of said belt portion (101) are lined with a fabric having a low coefficient of friction.

11. The back support garment according to one or more of the preceding claims, wherein said belt portion (101) has a thickness between 1 mm and 5 mm.

## Patentansprüche

1. Ein Rückenstützkleidungsstück für einen Träger, wobei das Kleidungsstück umfasst:
einen Kompressionshosenabschnitt (103), der so konfiguriert ist, dass er das Becken, die Beine und den Leistenbereich des Trägers stützt; und
einen Gürtelabschnitt (101), der so konfiguriert ist, dass er einen Lenden- und einen Bauchbereich des Trägers stützt, und der einen hinteren Abschnitt (105) umfasst, der so konfiguriert ist, dass er den Lendenabschnitt des Trägers überlagert und stützt,
wobei der Gürtelabschnitt (101) ein erstes Ende (113) und ein zweites Ende (114) aufweist, die lösbar aneinander befestigbar sind, um den Gürtelabschnitt (101) fest um die Taille des Trägers einstellbar zu sichern;
und wobei der Gürtelabschnitt (101) fest an dem Kompressionshosenabschnitt (103) angebracht ist und sich von diesem nach oben erstreckt, um den hinteren Abschnitt (105) mit dem Lendenbereich des Trägers auszurichten,
**dadurch gekennzeichnet, dass** die Rückenstützbekleidung eine Packungsstützstruktur (301) aufweist, die darin eine oder mehrere Heiß- oder Kaltpackungen (303) neben einer Innenfläche des Gürtelabschnitts (101) stützt,
wobei der Gürtelabschnitt (101) an dem Hosenabschnitt (103) so angebracht ist, dass der Packungsstützabschnitt (301) mit dem Lendenbereich des Trägers ausgerichtet wird und der temperaturübertragende Kontakt zwischen einer Innenfläche der Packungsstützstruktur (301), die an die Heiß- und Kaltpackungen (303) angrenzt, und dem Lendenbereich des Trägers aufrechterhalten wird, und wobei ein hinterer Abschnitt (107) des Kompressionshosenabschnitts (103) fest an dem hinteren Abschnitt (105) des Gürtelabschnitts (101) neben den Heiß- oder Kaltpackungen (303) befestigt ist.

2. Rückenstützkleidungsstück nach Anspruch 1, wobei der hintere Teil (105) des Gürtelteils (101) eine Versteifungsstruktur aufweist, die eine Struktur zur Aufnahme von Einsätzen (133) umfasst, die darin einen oder mehrere Einsätze (135) trägt, die so konfiguriert sind, dass sie den Rücken des Trägers weiter stützen, wobei die Einsätze (135) aus einem Material bestehen, das eine größere Biegefestigkeit aufweist als der hintere Teil (105) des Gürtelteils (101), wobei die Einsatzträgerstruktur (133) Schlitze zur Aufnahme der Einsätze (135) aufweist und die Einsätze (135) vom Träger durch Herausgleiten aus dem zugehörigen Schlitz entfernbar sind, und wobei die Einsätze (135) aus Kunststoff sind.

3. Rückenstützkleidungsstück nach Anspruch 1 oder 2, wobei der Kompressionshosenabschnitt (103) eine kurze Hose ist, die sich nicht tiefer als die Oberschenkel des Trägers erstreckt, oder der Kompressionshosenabschnitt (103) sich nach unten über die Knie des Trägers hinaus erstreckt.

4. Rückenstützkleidungsstück nach Anspruch 1, wobei das erste und zweite Ende (113, 114) des Gürtelabschnitts (101) in einem vorderen Abschnitt des Gürtelabschnitts (101) angeordnet sind.

5. Rückenstützkleidungsstück nach Anspruch 4, wobei das erste Ende (113) des Gürtelabschnitts (101) ein fest daran befestigtes Klettverschlussmaterial (116) aufweist und das zweite Ende (114) des Gürtelabschnitts ein komplementäres Klettverschlussmaterial (118) aufweist, das fest daran befestigt ist, so dass das erste Ende (113) lösbar und einstellbar an dem zweiten Ende (114) befestigt werden kann.

6. Das Rückenstützkleidungsstück gemäß einem oder mehreren der vorstehenden Ansprüche, wobei der Hosenabschnitt (103) einen vorderen Taillenbandabschnitt (115, 117) aufweist, der lösbar an einem Abschnitt (119, 121) des Gürtelabschnitts (101) befestigbar ist, um eine relative Position des Hosenabschnitts (103) zum Gürtelabschnitt (101) einzustellen.

7. Rückenstützkleidungsstück nach Anspruch 6, wobei der vordere Bundabschnitt (115, 117) des Hosenabschnitts (103) ein fest daran befestigtes Klettverschlussmaterial aufweist und der Abschnitt des Gürtelabschnitts (101) ein komplementäres Klettverschlussmaterial (119, 121) aufweist, das fest daran befestigt ist, um eine relative Position des Hosenabschnitts (103) zum Gürtelabschnitt (101) einzustellen.

8. Das Rückenstützkleidungsstück nach einem oder mehreren der vorstehenden Ansprüche, wobei der Gürtelabschnitt (101) aus einem atmungsaktiven und halbstarren Material hergestellt ist.

9. Rückenstützkleidungsstück nach einem oder mehreren der vorstehenden Ansprüche, wobei der Kompressionshosenabschnitt (103) aus einem Material hergestellt ist, das ein elastisches Gewebe enthält, so dass der Kompressionshosenabschnitt (103) den Körper des Trägers komprimiert, wenn er sich darauf befindet, wobei der Kompressionshosenabschnitt (103) aus einem Material hergestellt ist, das feuchtigkeitstransportierende Eigenschaften hat, und wobei der Kompressionshosenabschnitt (103) aus Nylongewebe hergestellt ist.

10. Rückenstützkleidungsstück nach einem oder mehreren der vorstehenden Ansprüche, wobei die Oberflächen des Gürtelteils (101) mit einem Gewebe mit einem niedrigen Reibungskoeffizienten gefüttert sind.

11. Das Rückenstützkleidungsstück nach einem oder mehreren der vorstehenden Ansprüche, wobei der Gürtelabschnitt (101) eine Dicke zwischen 1 mm und 5 mm aufweist.

## Revendications

1. Vêtement de soutien dorsal pour un porteur, ledit vêtement comprenant :
une partie pantalon de compression (103) qui est configurée pour soutenir le bassin, les jambes et la région de l'aine du porteur ; et
une partie ceinture (101) qui est configurée pour soutenir une région lombaire et une région abdominale du porteur, et incluant une partie arrière (105) configurée pour recouvrir et soutenir la partie lombaire du porteur ;
ladite partie ceinture (101) comprend une première extrémité (113) et une deuxième extrémité (114) qui peuvent être attachées de manière amovible l'une à l'autre de manière à fixer de manière réglable ladite partie ceinture (101) étroitement autour de la taille du porteur ;
dans lequel ladite partie ceinture (101) est attachée de manière fixe à ladite partie pantalon de compression (103) et s'étend vers le haut à partir de celle-ci, de manière à aligner la partie arrière (105) avec la région lombaire du porteur,
dans lequel le vêtement de soutien dorsal a une structure de support de sachet (301) supportant à l'intérieur de celle-ci un ou plusieurs sachets chauds ou froids (303)
**caractérisé en ce que** la structure de support de sachet (301) est adjacente à une surface interne de ladite partie ceinture (101),
ladite partie ceinture (101) étant attachée à ladite partie pantalon (103) de manière à aligner la partie de support de sachet (301) avec la région lombaire du porteur et maintenir un contact de transfert de température entre une surface interne de la structure de support de sachet (301) adjacente auxdits sachets chauds et froids (303) et la région lombaire du porteur, et
dans lequel une partie arrière (107) de ladite partie pantalon de compression (103) est fixée solidement à la partie arrière (105) de ladite partie ceinture (101) adjacente aux sachets chauds ou froids (303).

2. Vêtement de soutien dorsal selon la revendication 1, dans lequel ladite partie arrière (105) de la partie ceinture (101) inclut une structure de rigidification comprenant une structure de réception d'insert (133) supportant dans celle-ci un ou plusieurs inserts (135) qui sont configurés pour fournir un soutien supplémentaire au dos du porteur, lesdits inserts (135) étant composés de matériau ayant une résistance à la flexion supérieure à celle de la partie arrière (105) de la partie ceinture (101), dans lequel ladite structure de support d'insert (133) a des fentes recevant lesdits inserts (135), et lesdits inserts (135) pouvant être retirés par le porteur par glissement hors de la fente associée, et dans lequel lesdits inserts (135) sont en plastique.

3. Vêtement de soutien dorsal selon la revendication 1 ou 2, dans lequel ladite partie pantalon de compression (103) est un short ne s'étendant pas plus bas que les cuisses du porteur, ou ladite partie pantalon de compression (103) s'étend vers le bas au-delà des genoux du porteur.

4. Vêtement de soutien dorsal selon la revendication 1, dans lequel les première et deuxième extrémités (113, 114) de ladite partie ceinture (101) sont agencées dans une partie avant de ladite partie ceinture (101).

5. Vêtement de soutien dorsal selon la revendication 4, dans lequel ladite première extrémité (113) de ladite partie ceinture (101) a un matériau de fermeture à crochets et boucles (116) fixé solidement à celle-ci et ladite deuxième extrémité (114) de ladite partie ceinture a un matériau de fermeture à crochets et boucles complémentaire (118) fixé solidement à celle-ci de sorte que ladite première extrémité (113) peut être attachée de manière amovible et réglable à ladite deuxième extrémité (114).

6. Vêtement de soutien dorsal selon l'une ou plusieurs des revendications précédentes, dans lequel ladite partie pantalon (103) a une partie ceinture montée avant (115, 117) qui peut être attachée de manière amovible à une partie (119, 121) de ladite partie ceinture (101) pour régler une position relative de la partie pantalon (103) par rapport à la partie ceinture (101).

7. Vêtement de soutien dorsal selon la revendication 6, dans lequel ladite partie ceinture montée avant (115, 117) de ladite partie pantalon (103) a un tissu de fermeture à crochets et boucles fixé solidement à celle-ci et ladite partie de ladite partie ceinture (101) a un matériau de fermeture à crochets et boucles complémentaire (119, 121) fixé solidement à celle-ci pour régler une position relative de la partie pantalon (103) par rapport à la partie ceinture (101).

8. Vêtement de soutien dorsal selon l'une ou plusieurs des revendications précédentes, dans lequel ladite partie ceinture (101) est faite d'un matériau respirant et semi-rigide.

9. Vêtement de soutien dorsal selon l'une ou plusieurs des revendications précédentes, dans lequel ladite partie pantalon de compression (103) est faite d'un matériau contenant un tissu élastique de sorte que la partie pantalon de compression (103) comprime le corps du porteur lorsqu'il est présent sur celle-ci, dans lequel ladite partie pantalon de compression (103) est faite d'un matériau ayant des propriétés d'évacuation de l'humidité, et dans lequel ladite partie pantalon de compression (103) est faite d'un tissu en nylon.

10. Vêtement de soutien dorsal selon l'une ou plusieurs des revendications précédentes, dans lequel des surfaces de ladite partie ceinture (101) sont doublées d'un tissu ayant un faible coefficient de frottement.

11. Vêtement de soutien dorsal selon l'une ou plusieurs des revendications précédentes, dans lequel ladite partie ceinture (101) a une épaisseur comprise entre 1 mm et 5 mm.
